# EUROPEAN PATENT SPECIFICATION

(11) **EP 4 197 646 B1**
(45) Date of publication and mention of the grant of the patent: **05.08.2026**
(21) Application number: 21215761.4
(22) Date of filing: 17.12.2021
(51) Int. Cl.: B05B 12/00

(54) **A NEBULIZER WITH PLUME DETECTION**
VERNEBLER MIT FAHNENERKENNUNG
PULVÉRISATEUR AVEC DÉTECTION DE PANACHE

(43) Date of publication of application: 21.06.2023
(73) Proprietor: Stamford Devices Limited, H91 HE94 Galway (IE)
(72) Inventor: GREHAN, Joseph, H91 HE94 Galway (IE); DUFFY, Aidan, H91 HE94 Galway (IE); REDMOND, Anthony, H91 HE94 Galway (IE)
(74) Representative: Weldon O'Brien Ltd.

(56) References cited:
- US-A1- 2007 240 712
- US-A1- 2010 282 247
- US-A1- 2019 134 330
- US-B2- 6 744 046
- US-B2- 8 555 874

## Description

### Introduction

The present invention relates to nebulizers and their operation.

Known nebulizers include those of the type having a vibrating mesh or "aperture plate" which is vibrated by a drive such as a piezoelectric device. The piezoelectric device may be annular and mounted on a washer-shaped support for the aperture plate. Examples are those described in our PCT publications WO2012/046220 and WO2021/191160.

Further examples are described in documents US 2007/240712 A1, US 8 555 874 B, US 6 744 046 B2, US 2019/134330 A1 and US 2010/282247 A1.

The present invention addresses the problem of providing automatic sensing of operation of the nebulizer with reduced or no interference with its operation, and/or in a manner which is simple and/or inexpensive. For example, if a non-invasive sensing arrangement can be provided the requirement for re-validation of a nebulizer can be avoided.

### Summary of the Disclosure

The invention provides a nebulizer as set out in claim 1, and optional features are set out in claims 2-15.

### Detailed Description of the Invention

The invention will be more clearly understood from the following description of some embodiments thereof, given by way of example only with reference to the accompanying drawings in which:
Figs. 1 and 2 are perspective views of a nebulizer incorporating a sensor which monitors in real time operation of the nebulizer in terms of generation of aerosol directed into a conduit and into T-piece coupler, and Fig. 3 is a cross-sectional view of the aerosol generator;
Fig. 4 is a perspective view of another nebulizer, in this case incorporated in a vaccine dispensing station which dispenses a vaccine aerosol into a cup-shaped chamber for immediate inhalation by a user and there is real time monitoring of aerosol plume,
Fig. 5 is a perspective view of the nebulizer head of the nebulizer of Fig. 4, Fig. 6 is an exploded view of the head, and Fig. 7 is an enlarged perspective view of the head;
Fig. 8 is an image taken from a simulation showing the pattern of droplet formation from the nebulizer aperture plate, which is dome shaped with central and shoulder regions;
Fig. 9 is a set of images of droplet formation for help in understanding the method of operation of the sensor;
Figs. 10 and 11 are plots showing intensity of a signal picked up by the sensor, in which Fig. 11 shows a window provided by a filter; and
Figs. 12 and 13 are plots showing plume and no plume respectively.

The invention provides for very effective and non-invasive monitoring of plume generated by a vibrating mesh or aperture plate aerosol generator of a nebulizer. In one example the aerosol generator comprises a vibratable aperture plate, an annular support supporting the aperture plate, a vibration generator attached to the annular support, a power conductor for transferring power to the vibration generator, a downstream resilient seal mounted between a housing and the annular support on a side of the aperture plate opposed to the liquid supply reservoir, and an upstream resilient seal mounted between the annular support and the housing reservoir, and having an opening forming part of a throat over the aperture plate.

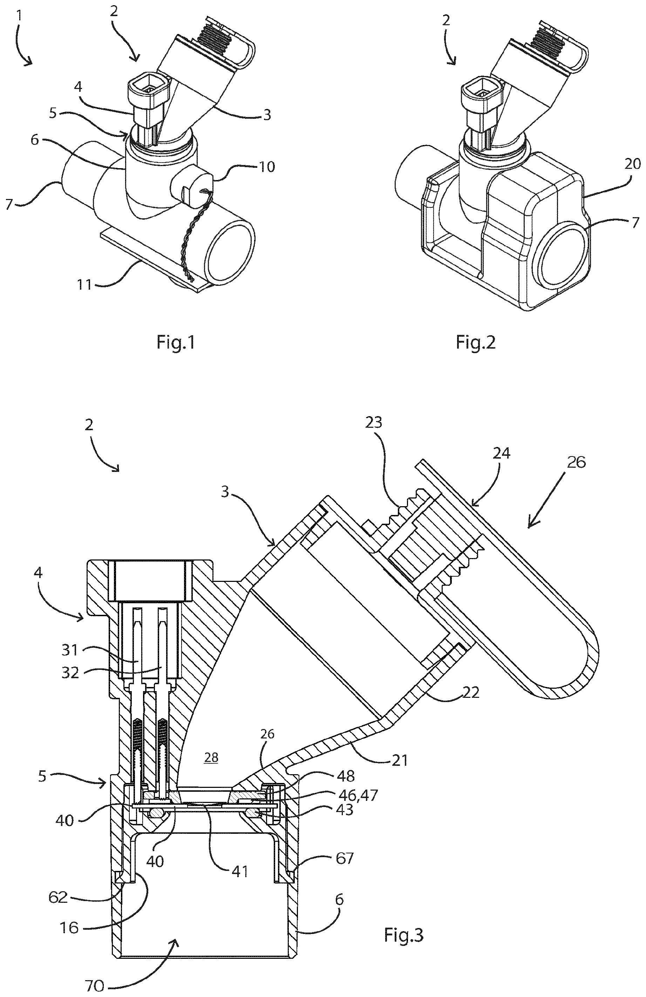

Figs. 1 and 2 are perspective views of a nebulizer 1 incorporating a sensor 10/11 which monitors in real time operation of the nebulizer in terms of generation of aerosol directed into a conduit 6 and into a T-piece coupler 6. The nebulizer 1 comprises a housing 2 with a funnel-shaped reservoir 3 mounted at an angle to a longitudinal axis of the aperture plate aerosol generator assembly 5, and power is provided by spring pins in a pin housing 4, The aerosol generator 5 is on-axis with the outlet conduit 6, which is fitted to a T-piece conduit 7. The sensor comprises an acoustic transducer 10 linked with a drive and sensor circuit 11. The acoustic transducer is in this example a piezoelectric transducer to convert ultrasound energy into electric energy, configured for pickup in a band centred on 60kHz.

The nebulizer 1 further comprises a housing 20 surrounding the sensor, in which a lower part supports the circuit 11 and a top part supports and surrounds the acoustic transducer 10.

Referring specifically to Fig. 3 the nebulizer also comprises a retainer 16 supporting the aerosol generator 5 in the integral housing body 2, a reservoir funnel-shaped lower part 21, a reservoir tubular top part 22, a liquid supply chamber cap 26, a cap tubular opening 23, and a cap silicone plug 24. There are conducting pins 31 and 32 extending through the pin support housing part 4. Aerosol is generated by an aperture plate 41, in this case having apertures each having an outlet opening diameter in the range of 1µm to 10µm, and in one preferred example about 2µm to 3µm. In this case the AP 41 has a 19.63mm² mesh area and an aperture density of 50 holes/mm², preferably in the range of 30 holes/mm² to 70 holes/mm². There is a top gasket 48 acting as a resilient annular seal between the housing 2 and the aerosol generator assembly 5.

The reservoir top part 22 is in fluid communication with the inclined funnel-shaped lower part 21 for delivering liquid onto the aperture plate ("AP") 41. The aerosol delivery tubular outlet 6 is below the AP, is integral with the reservoir 3, and is co-axial with the AP 41. The axis of the funnel 21 is inclined to the AP 41 axis. This allows use of the nebulizer at a wide range of orientations with gravity fall of the liquid onto the AP.

The conducting pins 31 and 32 are for conducting power to a piezoelectric vibration generator 46 of the aerosol generator 5, and are retained within, and guided by, the pin housing part 4 of the housing body 2.

The retainer 16 is for carrying the aerosol generator assembly 5, pressed against a lower surface of the reservoir 3. The aerosol generator assembly 5 comprises, from top down, an upstream seal namely the gasket 48, the piezoelectric vibration generator ("piezo") 46, an adhesive ring 47 under the piezo 46, the aperture plate 41, a braze ring on the rim of the AP 41, an annular washer-shaped aerosol generator support ("washer") 40, and a downstream annular resilient seal, in this case an O-ring 43. This assembly is supported by the retainer 16 and is surrounded by the aerosol delivery tube 6. The adhesive ring 47 bonds the (annular) piezo 46 to the washer 40, attaching the piezo to the washer in a manner which conducts electrical power to the underside of the piezo 46. A braze ring is an integral part of the rim of the aperture plate ("AP") 41 and the internal rim of the washer 40, attaching the AP to the washer. In other examples the attachment of the AP may be bonded rather than brazed.

This example involves an electroformed aperture plate 41 with "hourglass" shaped apertures. However, in other examples the aperture plate may be formed by photo-defined technology as described in our prior patent specification nos. WO2012/092163 or WO2013/186031. There may be a reservoir layer of liquid supply cavities leading into the aerosol-forming apertures, formed in a manner for example as described in these specifications, and these may have a diameter in the range of 20µm to 400µm. Each cavity overlies multiple aerosol-forming apertures.

The retainer 16 is configured so as to securely support and contain the components of the aerosol generator assembly 5. It has a circumferential wall with a top rim and a pair of lower depending legs 62 with toes which snap fit as clips into corresponding recesses 67 of the aerosol outlet tube 6. At the upper end, a series of circumferentially extending elongate tabs define the outer surface of a circular seat for the gasket 48.

The retainer 16 also forms an annular seat for the downstream O-ring 43. The washer 40 is supported underneath by the O-ring 43 housed within the groove 68 of the retainer 6, the washer 40 resting on the O-ring 43 and itself supporting the piezo 46 adhered to the washer 40 top surface. The AP 41 is attached by the braze ring 42 to the washer 40.

The liquid supply reservoir funnel 21 has an internal tapered surface 26 inclined inwardly towards the AP 41, defining a throat 28 over the AP together with the gasket inner surface. The latter is not overhung by the housing, forming a continuation of the housing surface. By having two components, the gasket and the housing forming a reservoir shape leading to the aperture plate, it is convenient and versatile to provide a combination of surface types to optimise liquid flow to the AP, with minimization of bubble formation.

As is described in more detail below, as the aperture plate 41 is driven to vibrate, in one example at a frequency of 128kHz, and droplets having a size in the range of 2µm to 6µm break off the lower surface of the plate. On average it takes more than one and less than three vibrations to provide a droplet, and so the frequency of droplet formation is less than the AP drive frequency. The flow of the droplets gives rise to a characteristic sound at a frequency which is less than the AP drive frequency, and the transducer 10 picks up this acoustic signal. It is tuned to a number of frequencies around a value of half of the drive frequency, in this case 64kHz. The aerosol droplets move vertically downwardly within the conduit 6 from the AP 41 in a space indicated by 70 in Fig. 3, and the transducer 10 picks up the acoustic signal via the tubular wall 6. The sensing is therefore in a non-invasive manner, not affecting the aerosol generation.

### Alternative Nebulizer

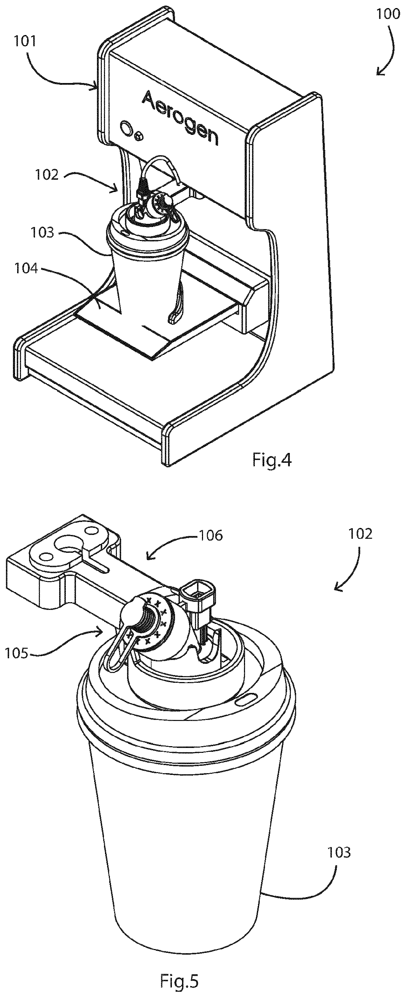

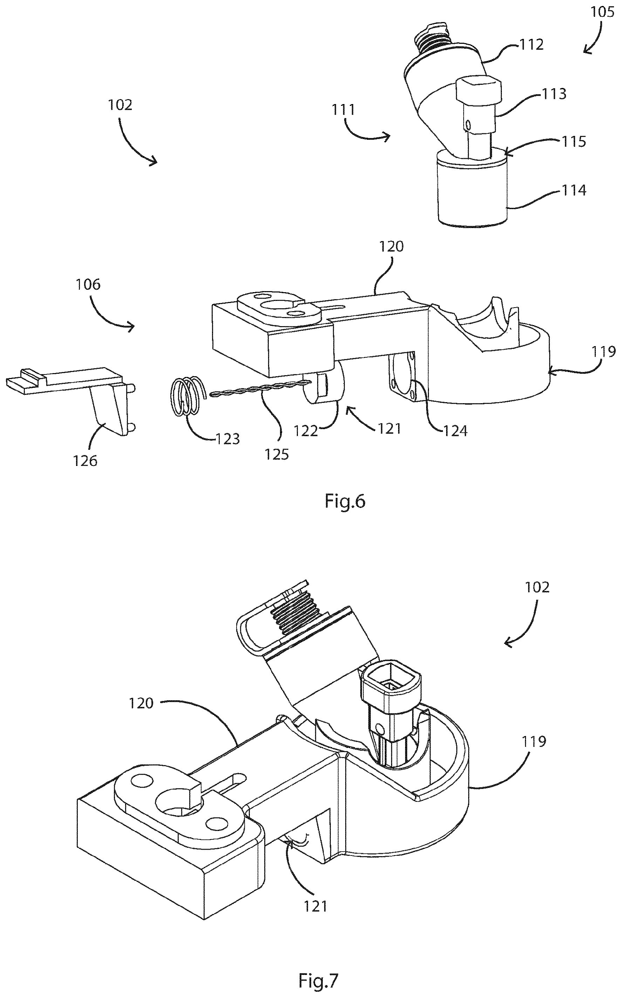

Fig. 4 is a perspective view of another nebulizer, 100, in this case having a housing 101 supporting a nebulizer head 102 for being incorporated in a vaccine dispensing station which dispenses a vaccine aerosol into a cup-shaped chamber 103 supported on a platform 104. The dispensed vaccine aerosol in the cup 103 is for immediate inhalation by a user and there is real time monitoring of aerosol plume. Fig. 5 is an enlarged perspective view of the head 102, and as shown in the exploded view of Fig. 6, the head 102 comprises an aerosol generator 105 and a support 106. The aerosol generator 105 comprises a tilted funnel-shaped reservoir 112, a housing 113 to one side for sprung conducting pins for transfer of power to the annular piezoelectric element, an aerosol generator core 115 with a vibrating aperture plate, and a tubular outlet conduit 114 which is co-axial with the aperture plate.

The support 106 comprises a ring 119 at the end of an arm 120, the ring 119 being configured to surround the tubular outlet 114, and it acts as a housing for an acoustic sensor 121 having a cylindrical housing 122 for an acoustic transducer at its distal end (nearest the conduit 114) and a drive and signal circuit, with a power lead 125 and a helical spring 123. The sensor housing 122 is held in a chamber 124 of the ring 119, the chamber guiding the sensor into position abutting the outlet conduit 114 when the conduit is encompassed by the ring 119. The sensor components, namely the sensor element 122, the helical spring 123, and the power lead 125 are retained by a plastics clip 126.

The aerosol generator assembly of the nebulizer 100 is the same as that of the nebulizer 1. The manner of operation of the sensors 10/11 and 22 are the same, both being located in contact with the outlet conduit through which the generated aerosol droplets flow. It is envisaged, though not as effective, to position the transducer spaced from the conduit wall by a distance in the range of 0.5mm to 5mm in other examples.

The nebulizer 100 is suited to dispense aerosol of a medication such as a vaccine into a succession of chambers 103. In one example this is in a vaccine station for high-volume dispensing of vaccines with successive operations of filling the chambers 103. In this case the sensor detection of plume is particularly advantageous because it allows control at the delivery end of the nebulizer, allowing accurate control of when a chamber is brought into registry with the nebuliser outlet conduit for consistent dispensing into the chambers 103.

Advantageously, the controller is configured to drive the aperture plate 41 to provide a single dose of aerosol per chamber 103, with the outlet conduit114 engaging the aerosol chamber 103 and in which the sensor 121 provides feedback of presence or absence of an aerosol plume. The sensing does not depend on an upstream condition such as presence of liquid on the aperture plate, the plum e absence or presence providing clear feedback of start and end of a single discrete dispensing operation.

Advantageously, the controller is configured to provide a series of doses to a series in this manner to chambers (103) which are presented to the nebulizer, wither manually or automatically by a handling system. It determines when the aerosol has been fully delivered into a chamber when the detector detects that there is no plume.

The method of operation of the nebulizer 100 is particularly advantageous for determining plume presence or absence according to the sensor detecting an acoustic signal from the space (70) downstream of the aperture plate. It is particularly advantageous where there are steps of providing a series of aerosol chambers to the nebulizer, the controller operating to provide aerosol to each in a series of discrete operations, and in which completion of each dispensing operation is determined when there is little or no plume detected by the sensor.

In one particular example the liquid is a vaccine, and each chamber 103 is for inhalation of a single vaccine dose by a patient.

### Droplet Formation Mechanism and Plume Monitoring

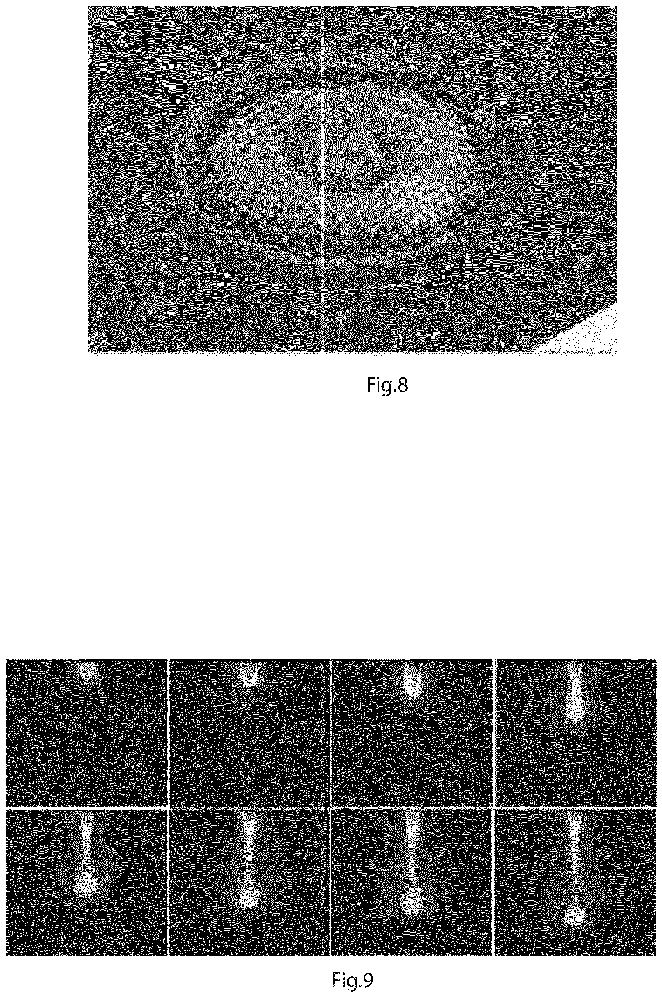

Fig. 8 is an image taken from a simulation showing the pattern of droplet formation from the aperture plate 41 which is dome shaped, with central and shoulder regions. The greatest density is in the centre and the second greatest density is in an annular region around the centre, corresponding to the lowermost point of the dome-shaped AP 41, and the curved region close to the flange or rim which engages the support washer. The droplets shown in the pattern of Fig. 8 are the majority of the droplets which give rise to the acoustic signal. Maximum displacement of the plate 41 occurs in the centre region. The mode shape/pattern of vibration is called mode 0,2.

Fig. 9 is a set of images of droplet formation for help in understanding the method of operation of the sensor. The droplet formation takes a time in the range of 1 to three plate vibrations on average.

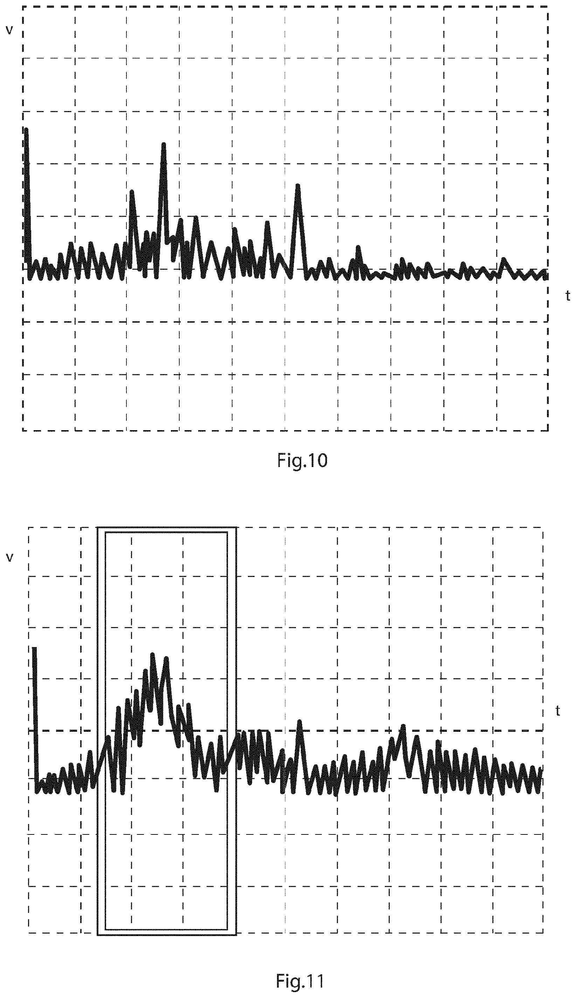

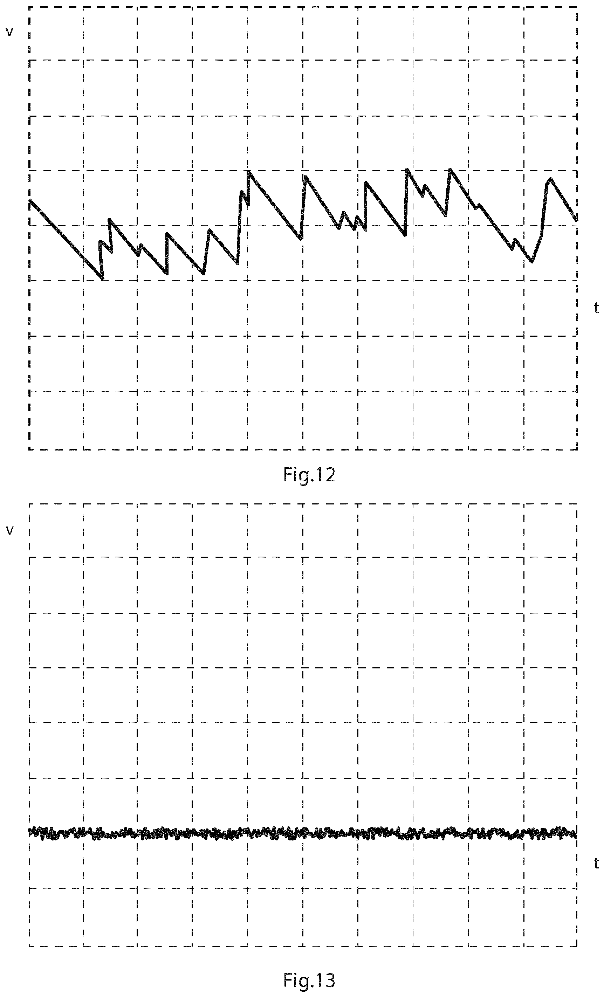

Figs. 10 and 11 are plots showing intensity of a signal picked up by the sensor, in which Fig. 11 shows a window provided by a filter. The area under the curve is computed to provide a value representative of the aerosol droplet plume side (number of droplets/droplet flow rate). There is a small plume for the situation of Fig. 10, and a large one for that of Fig. 11. Figs. 12 and 13 are also plots representing acoustic signal intensity vs. time, Fig. 12 representing a plume, and Fig. 13 representing no plume.

The sensor in positioned on the outlet of the nebulizer The signal strength increases with distance from the AP 41 due to the plume diverging as it leaves the plate. The sensor is preferably located in the range of 1 mm to 20 mm from the aperture plate as measured along a longitudinal axis of the aperture plate.

The signal that is picked up by the sensor is developed as a result of how the droplets are formed. The movement of the plate 41 and its mode shape determines how a droplet is formed, the majority of droplets are formed from the centre and the shoulder area as shown in Fig. 8. The droplet formation may take a number of cycles before a droplet exits the plate as shown in Fig. 9 and is somewhat chaotic in nature.

The sensor monitors delivery of liquid drug through detection of a unique acoustic signature of when delivering dose compared to the signature when delivery has ceased. During operation, the nebulizer is driven at a drive frequency of at 128 kHz. In addition to the output at the operating frequency, a small signal can be observed at sub-harmonic of the operating frequencies during delivery. The sensor of one example functions by monitoring a signal at 64 kilohertz range and analyzing the output for a distinct signature that is only present during delivery. In general, it is preferred that the transducer picks up signals in the range of one third to two-thirds of the drive frequency, because the droplets are primarily formed for one to three AP vibrations.

This signal is a by-product of the droplet formation and also partially due to energy transfer through the liquid. Droplets are not always created on each individual plate cycles. Droplet breakoff can also occur on every second cycle producing a unique output at half the operating frequency i.e. 64kHz. Acoustic energy from the vibrating plate will transfer through the liquid, where it is picked up on the outer surface of the device as an acoustic signal.

We have found the largest increase in sound intensity occurs in the 64 kHz frequency band when the nebulizer is generating a plume with the AP driven at 128kHz. The sensor has a hardware element to pickup and amplify the very small signal and a software element to process the signal and make the determination of plate status. Importantly, the sensor is not directly monitoring liquid presence on the AP, rather presence of a plume on the opposed (downstream) side of the AP.

The detected acoustic signal is amplified, using a two-stage amplifier to increase the intensity. The sensor circuit removes the unwanted frequencies using a band-pass filter, to leave only the area indicated by the box in Fig. 11. The overall frequency domain shown in the plot is 0-200kHz, and the boxed area is a 30kHz band, centred around 64kHz. The final hardware stage is a peak detector circuit that tracks the highest intensity of the signal.

The signal from the amplifier stage, in the time domain, looks like the plot of Figs. 12 and 13. The software monitors the peaks and troughs over a period of time and determines the level of disturbance from the plate when the plume is being generated. When the plume finishes the output signal is about 5 times smaller than the delivery signal (1.02V verses 0.2V), the no-plume situation being shown in Fig. 13. Without the filter stage, the noise produced from the drive signal alone would be the dominant signal, and will appear when both the plume is present or not.

### Advantages

The invention provides a simple and low-cost method of detecting delivery. The nebulizer delivery is not altered in any way, therefore there is no concern for invalidating a previously approved system. The sensing is not affected by rainout of liquid on the sidewall of the nebulizer as is the case with any visual system (visual detection systems require continuous cleaning).

The sensor size is very small (approximately 10mm in diameter) and can be placed close to the nebulizer body, with minimal interference.

The sensor can be an ancillary product that can be added to any existing system. It can be used for bespoke delivery stations where delivery status information is a requirement. It may be added to the T-piece element in a standard Ventilator circuit, as shown in Figs. 1 and 2. For example, it can operate as a standalone battery-operated device providing visual feedback or RF communication to external controller.

In other embodiments the transducer could be distal from the nebuliser and rely on the material properties of a T-piece or other coupler to transmit the signal to the transducer. It has been shown that a rigid acrylic sheet with a hole to accommodate a press fit insertion of the transducer can facilitate this. The T-piece could incorporate the necessary geometry to locate the PCB, the battery, the transducer and light guide/illumination features.

In the examples shown the transducer is in direct contact with the body of the nebuliser, and in some examples a compressed spring provides a constant pressure between the contact surfaces to ensure good and consistent transfer of energy from the nebuliser body to the transducer. The transducer is primarily only making contact with the nebuliser body and the spring. In an alternative embodiment the transducer would be rigidly coupled to the bracket, and the bracket would serve as an energy transfer medium between the nebulizer body and the transducer.

The sensor support is configured to position the transducer at the 'lowest' point on the body, furthest from the AP whilst still leaving a portion (~4.5mm) of the ISO port 6/114 protruding such that it can interface with the 22mm hole in the coupler for delivery of the aerosol.

Components of embodiments can be employed in other embodiments in a manner as would be understood by a person of ordinary skill in the art. The invention is not limited to the embodiments described but may be varied in construction and detail within the scope of the claims. For example, the liquid may be delivered to the aperture plate by a conduit. In one such example the liquid may be pumped. In another example the liquid is delivered by capillary action, with the conduit being sized for capillary flow.

## Claims

1. A nebulizer comprising:
an aerosol generator (1) comprising a vibratable aperture plate (41) with apertures having a size in the range of 1µm to 10µm,
means (3) to deliver a liquid to the aperture plate,
a controller with a drive circuit to cause the aperture plate (41) to vibrate at a drive frequency to cause droplets to separate from a downstream side of the aperture plate,
an outlet conduit (6, 114) for flow of droplets from the aperture plate,
**characterized in that** the nebulizer further comprises:
a sensor comprising an acoustic transducer (10, 122) arranged to pick up an acoustic signal downstream of said aperture plate, said signal being representative of an aerosol droplet plume, and
a sensor controller (20) with a digital data processor to analyse said signal to provide an output representative of a plume;
wherein:
the sensor (10, 11, 121) is arranged to pick up an acoustic signal at or adjacent said outlet conduit, and the acoustic transducer is located adjacent or in contact with an outer surface of said conduit (6, 114), and
the sensor is configured to monitor an acoustic signal in a frequency range which is lower than the drive frequency.

2. A nebulizer as claimed in claim 1, wherein the sensor (10, 11, 121) is located in the range of 1 mm to 20 mm from the aperture plate as measured along a longitudinal axis of the aperture plate.

3. A nebulizer as claimed in claim 2, wherein the sensor (10, 11, 121) is configured to monitor an acoustic signal in a frequency band in the range of one third to two thirds of the drive frequency.

4. A nebulizer as claimed in any preceding claim, wherein the processor is configured to automatically determine area under a curve of acoustic intensity and time for one or more transducer pickup frequencies.

5. A nebulizer as claimed in claim 4, wherein the intensity is represented by voltage.

6. A nebulizer as claimed in any preceding claim, wherein the transducer is pressed (123) against an outer surface of the conduit (114).

7. A nebulizer as claimed in any preceding claim, wherein the sensor is at least partially housed by a housing (20, 119) which is removably engageable with said conduit.

8. A nebulizer as claimed in claim 7, wherein the housing (119) surrounds at least part of the outlet conduit.

9. A nebulizer as claimed in claim 8, wherein the housing (20, 119) supports the aerosol generator.

10. A nebulizer as claimed in any preceding claim, wherein the aperture plate is attached to an internal rim of a washer-shaped support (40), and the nebulizer comprises an upstream annular seal (48) between the reservoir (3) and the support, and a downstream annular seal (43) between the support and a housing component.

11. A nebulizer as claimed in any claim 10, wherein the housing component comprises a retainer (16) which fits into the outlet conduit (6) and is arranged to press the downstream annular seal towards the support.

12. A nebulizer as claimed in any preceding claim, further comprising a cap (26) for the reservoir, arranged to cover an opening of the reservoir.

13. A nebulizer as claimed in any preceding claim, further comprising a support (104) for a chamber (103), and the controller is configured to drive the aperture plate to provide a single dose of aerosol per chamber, in which the outlet conduit engages an aerosol chamber and in which the sensor provides feedback of presence or absence of an aerosol plume.

14. A nebulizer as claimed in claim 13, wherein the controller is configured to provide a series of doses to a series of chambers (103) which are presented to the nebulizer, and to determine when the aerosol has been fully delivered into a chamber when the detector detects that there is no plume.

15. A nebulizer as claimed in any preceding claim, wherein the liquid delivery means comprises a reservoir (3) over the aperture plate.

## Patentansprüche

1. Vernebler, umfassend:
einen Aerosolerzeuger (1), umfassend eine vibrierfähige Lochplatte (41) mit Löchern, die eine Größe im Bereich von 1 µm bis 10 µm aufweisen,
Mittel (3) zum Zuführen einer Flüssigkeit zu der Lochplatte,
eine Steuereinheit mit einer Treiberschaltung, um die Lochplatte (41) zu veranlassen, mit einer Treiberfrequenz zu vibrieren, um Tröpfchen zu veranlassen, sich von einer stromabwärtigen Seite abzulösen,
eine Auslassleitung (6, 114) für einen Strom von Tröpfchen von der Lochplatte,
**dadurch gekennzeichnet, dass** der Vernebler ferner Folgendes umfasst:
einen Sensor, umfassend einen akustischen Wandler (10, 122), der dazu eingerichtet ist, ein akustisches Signal stromabwärts der Lochplatte zu empfangen, wobei das Signal für eine Aerosoltröpfchenfahne repräsentativ ist, und
eine Sensorsteuereinheit (20) mit einem digitalen Datenprozessor zum Analysieren des Signals, um einen eine Fahne repräsentierenden Ausgang bereitzustellen;
wobei:
der Sensor (10, 11, 121) dazu eingerichtet ist, ein akustisches Signal an der Auslassleitung oder dieser benachbart zu empfangen und der akustische Wandler einer Außenoberfläche der Leitung (6, 114) benachbart oder in Kontakt damit angeordnet ist und
der Sensor dazu konfiguriert ist, ein akustisches Signal in einem Frequenzbereich zu überwachen, der niedriger als die Treiberfrequenz ist.

2. Vernebler nach Anspruch 1, wobei der Sensor (10, 11, 121) im Bereich von 1 mm bis 20 mm von der Lochplatte, gemessen entlang einer Längsachse der Lochplatte, angeordnet ist.

3. Vernebler nach Anspruch 2, wobei der Sensor (10, 11, 121) dazu konfiguriert ist, ein akustisches Signal in einem Frequenzband im Bereich von einem Drittel bis zwei Dritteln der Treiberfrequenz zu überwachen.

4. Vernebler nach einem der vorangehenden Ansprüche, wobei der Prozessor dazu konfiguriert ist, eine Fläche unter einer Kurve akustischer Intensität und Zeit für eine oder mehrere Wandlerempfangsfrequenzen automatisch zu bestimmen.

5. Vernebler nach Anspruch 4, wobei die Intensität durch eine Spannung repräsentiert wird.

6. Vernebler nach einem der vorangehenden Ansprüche, wobei der Wandler an eine Außenoberfläche (114) der Leitung gedrückt (123) wird.

7. Vernebler nach einem der vorangehenden Ansprüche, wobei der Sensor mindestens teilweise von einem Gehäuse (20, 119) untergebracht ist, das mit der Leitung entfernbar eingriffsfähig ist.

8. Vernebler nach Anspruch 7, wobei das Gehäuse (119) mindestens einen Teil der Auslassleitung umgibt.

9. Vernebler nach Anspruch 8, wobei das Gehäuse (20, 119) den Aerosolerzeuger trägt.

10. Vernebler nach einem der vorangehenden Ansprüche, wobei die Lochplatte an einem inneren Rand einer unterlegscheibenförmigen Halterung (40) angebracht ist und der Vernebler eine stromaufwärtige ringförmige Dichtung (48) zwischen dem Behälter (3) und der Halterung und eine stromabwärtige ringförmige Dichtung (43) zwischen der Halterung und einer Gehäusekomponente umfasst.

11. Vernebler nach Anspruch 10, wobei die Gehäusekomponente einen Halter (16) umfasst, der in die Auslassleitung (6) passt und dazu eingerichtet ist, die stromabwärtige ringförmige Dichtung in Richtung der Halterung zu drücken.

12. Vernebler nach einem der vorangehenden Ansprüche, ferner umfassend eine Kappe (26) für den Behälter, die dazu eingerichtet ist, eine Öffnung des Behälters abzudecken.

13. Vernebler nach einem der vorangehenden Ansprüche, ferner umfassend eine Halterung (104) für eine Kammer (103), wobei die Steuereinheit dazu konfiguriert ist, die Lochplatte anzutreiben, um eine einzelne Dosis von Aerosol pro Kammer bereitzustellen, wobei sich die Auslassleitung mit einer Aerosolkammer im Eingriff befindet und wobei der Sensor eine Rückmeldung über Anwesenheit oder Abwesenheit einer Aerosolfahne bereitstellt.

14. Vernebler nach Anspruch 13, wobei die Steuereinheit dazu konfiguriert ist, eine Reihe von Dosen einer Reihe von Kammern (103), die dem Vernebler dargeboten werden, bereitzustellen, und zu bestimmen, wann das Aerosol vollständig in eine Kammer zugeführt worden ist, wenn der Detektor erkennt, dass keine Fahne vorhanden ist.

15. Vernebler nach einem der vorangehenden Ansprüche, wobei das Flüssigkeitszuführmittel einen Behälter (3) über der Lochplatte umfasst.

## Revendications

1. Un nébuliseur comprenant :
un générateur d'aérosol (1) comprenant une plaque à ouvertures capable de vibrer (41) avec des ouvertures ayant une taille dans la plage de 1 µm à 10 µm,
des moyens (3) pour délivrer un liquide à la plaque à ouvertures,
un dispositif de commande avec un circuit d'entraînement pour amener la plaque à ouvertures (41) à vibrer à une fréquence d'entraînement pour provoquer la séparation de gouttelettes à partir d'un côté aval de la plaque à ouvertures,
un conduit de sortie (6, 114) pour l'écoulement de gouttelettes à partir de la plaque à ouvertures
**caractérisé en ce que** le nébuliseur comprend en outre :
un capteur comprenant un transducteur acoustique (10, 122) agencé pour capter un signal acoustique en aval de ladite plaque à ouvertures, ledit signal étant représentatif d'un panache de gouttelettes d'aérosol, et
un dispositif de commande de capteur (20) avec un processeur de données numériques pour analyser ledit signal afin de fournir une sortie représentative d'un panache ;
dans lequel :
le capteur (10, 11, 121) est agencé pour capter un signal acoustique au niveau dudit conduit de sortie ou adjacent audit conduit de sortie, et le transducteur acoustique est situé adjacent ou en contact avec une surface externe dudit conduit (6, 114), et
le capteur est configuré pour surveiller un signal acoustique dans une plage de fréquence qui est inférieure à la fréquence d'entraînement.

2. Nébuliseur selon la revendication 1, dans lequel le capteur (10, 11, 121) est situé dans la plage de 1 mm à 20 mm de la plaque à ouvertures comme mesuré le long d'un axe longitudinal de la plaque à ouvertures.

3. Nébuliseur selon la revendication 2, dans lequel le capteur (10, 11, 121) est configuré pour surveiller un signal acoustique dans une bande de fréquence dans la plage d'un tiers à deux tiers de la fréquence d'entraînement.

4. Nébuliseur selon l'une quelconque des revendications précédentes, dans lequel le processeur est configuré pour déterminer automatiquement l'aire sous une courbe d'intensité acoustique et de temps pour une ou plusieurs fréquences de captage de transducteur.

5. Nébuliseur selon la revendication 4, dans lequel l'intensité est représentée par une tension.

6. Nébuliseur selon l'une quelconque des revendications précédentes, dans lequel le transducteur est appuyé (123) contre une surface externe du conduit (114).

7. Nébuliseur selon l'une quelconque des revendications précédentes, dans lequel le capteur est au moins partiellement logé dans un logement (20, 119) qui peut être mis en prise de manière amovible avec ledit conduit.

8. Nébuliseur selon la revendication 7, dans lequel le logement (119) entoure au moins une partie du conduit de sortie.

9. Nébuliseur selon la revendication 8, dans lequel le logement (20, 119) supporte le générateur d'aérosol.

10. Nébuliseur selon l'une quelconque des revendications précédentes, dans lequel la plaque à ouvertures est attachée à un rebord interne d'un support en forme de rondelle (40), et le nébuliseur comprend un joint annulaire amont (48) entre le réservoir (3) et le support, et un joint annulaire aval (43) entre le support et un composant de logement.

11. Nébuliseur selon l'une quelconque revendication 10, dans lequel le composant de logement comprend un élément de retenue (16) qui s'ajuste jusque dans le conduit de sortie (6) et est agencé pour appuyer le joint annulaire aval vers le support.

12. Nébuliseur selon l'une quelconque des revendications précédentes, comprenant en outre un capuchon (26) pour le réservoir, agencé pour couvrir une ouverture du réservoir.

13. Nébuliseur selon l'une quelconque des revendications précédentes, comprenant en outre un support (104) pour une chambre (103), et le dispositif de commande est configuré pour amener la plaque à ouvertures à fournir une dose unique d'aérosol par chambre, dans lequel le conduit de sortie entre en prise avec une chambre d'aérosol et dans lequel le capteur fournit une rétroaction de présence ou d'absence d'un panache d'aérosol.

14. Nébuliseur selon la revendication 13, dans lequel le dispositif de commande est configuré pour fournir une série de doses à une série de chambres (103) qui sont présentées au nébuliseur, et pour déterminer quand l'aérosol a été complètement délivré dans une chambre lorsque le détecteur détecte qu'il n'y a pas de panache.

15. Nébuliseur selon l'une quelconque des revendications précédentes, dans lequel le moyen de délivrance de liquide comprend un réservoir (3) sur la plaque à ouvertures.
